# EUROPÄISCHE PATENTANMELDUNG

(11) **EP 4 053 113 A1**
(43) Veröffentlichungstag der Anmeldung: **07.09.2022**
(21) Anmeldenummer: 21160527.4
(22) Anmeldetag: 03.03.2021
(51) Int. Cl.: C07D 307/32

(54) **VERBESSERTES VERFAHREN ZUR HERSTELLUNG VON ALKY-4-OXO-TETRAHYDROFURAN-2,3-DICARBOXYLATE**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein neues Verfahren zur Herstellung von Alkyl-4-oxotetrahydrofuran-2-carboxylat (**I**).

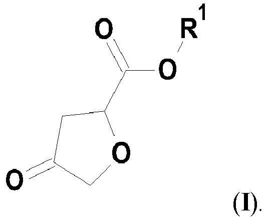

## Beschreibung

Die vorliegende Erfindung betrifft ein neues Verfahren zur Herstellung von Alkyl-4-oxotetrahydrofuran-2-carboxylat (**I**).

Methyl-4-oxotetrahydrofuran-2-carboxylat der Formel (**I**) ist eine wichtige Vorstufe von agrochemischen (vgl. WO2018/228985) Wirkstoffen.

Die Synthese von Methyl-4-oxotetrahydrofuran-2-carboxylat der Formel (**I**) ist bekannt, z.B. aus Helv. Chim. Acta 1959, 1177 und WO 2016/205633. Jedoch sind ausgehend von Dimethyl(*Z*)-but-endioate drei Reaktionsschritte notwendig, um Methyl-4-oxotetrahydrofuran-2-carboxylat der Formel (**I**) herzustellen, was mit einem Ausbeuteverlust einhergeht. Des Weiteren sind die Reagenzien, die im Stand der Technik verwendet werden, (z.B. Natriumpulver, NaH, TMSCHN₂, CH₂N₂) nicht für eine großtechnische Synthese geeignet, da der sichere Umgang mit diesen Chemikalien im großen Maßstab schwierig ist oder sie sehr toxisch sind.

Im Lichte des zuvor beschriebenen Standes der Technik liegt der vorliegenden Erfindung die Aufgabe zugrunde, ein Verfahren zur Herstellung der Verbindungen der allgemeinen Formel (**I**) ausgehend von Verbindungen der allgemeinen Formel (**II**) und (**III**) in nur zwei Reaktionsschritten zu entwickeln, das auch für die Produktion in großem Maßstab geeignet ist.

Die zuvor beschriebene Aufgabe wird gelöst durch ein Verfahren zur Herstellung von Verbindungen der allgemeinen Formel (**I**) 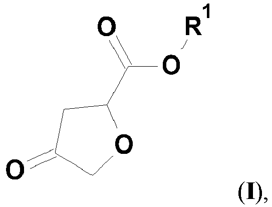 worin
- R¹: (C₁-C₄)-Alkyl ist,
dadurch gekennzeichnet, dass Verbindungen der allgemeinen Formel (**II**) 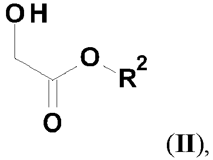 worin
- R²: (C₁-C₄)-Alkyl ist,
mit Verbindungen der allgemeinen Formel (**III**) 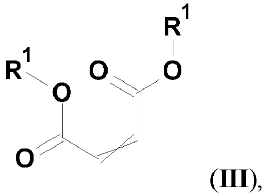 worin R¹ wie oben definiert ist,
durch Zugabe von **M**O-**Z** in **Z**-OH,
worin
- M: für ein Alkalimetallion steht und
- Z: (C₁-C₄)-Alkyl, ausgenommen *t*Butyl, ist,

Zyklisierungsprodukte mit der allgemeinen Formel (**IV**) ergeben 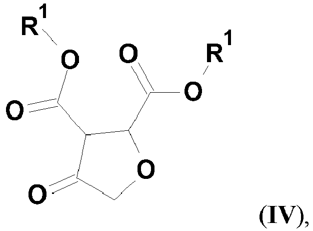 worin R¹ wie oben definiert ist,
welche unter nicht hydrolytischen Bedingungen eine Dealkoxycarbonylierung unterlaufen und zu Verbindungen der allgemeinen Formel (**I**) reagieren.

Bevorzugte Restedefinitionen für die Verbindungen der allgemeinen Formeln (**I**), (**II**), (**III**), (**IV**), **M**O-**Z** und **Z**-OH sind die folgenden:
- R¹: steht für Ethyl oder Methyl,
- R²: steht für Ethyl oder Methyl,
- M: steht für Natrium oder Kalium,
- Z: steht für Ethyl oder Methyl.

Besonders bevorzugte Restedefinitionen für die Verbindungen der allgemeinen Formeln (**I**), (**II**), (**III**), (**IV**), **M**O-**Z** und **Z**-OH sind die folgenden:
- R¹: steht für Methyl,
- R²: steht für Methyl,
- M: steht für Natrium,
- Z: steht für Methyl.

### Erläuterung des Verfahrens

Die Reaktionsbedingungen zur Herstellung von Verbindungen der allgemeinen Formel (**I**) werden im Folgenden im Detail erläutert.

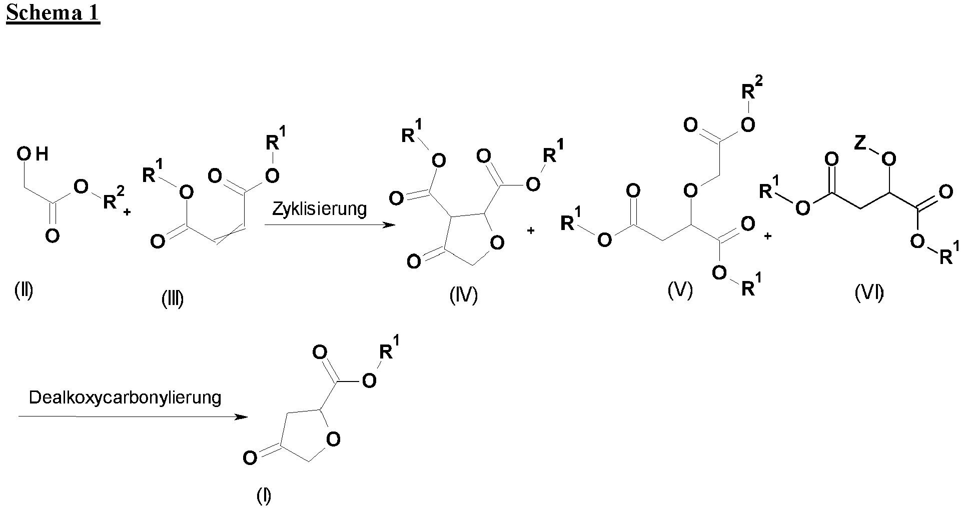

Die Verbindungen der allgemeinen Formel (**II**) reagieren mit Verbindungen der allgemeinen Formel (**III**) in Anwesenheit von **M**O-**Z** in **Z**-OH zu Zyklisierungsprodukten mit der allgemeinen Formel (**IV**), welche unter nicht hydrolytischen Bedingungen eine Dealkoxycarbonylierung unterlaufen und zu Verbindungen der allgemeinen Formel (**I**) reagieren.

Während der Zyklisierung können neben dem eigentlichen Produkt, den Verbindungen der allgemeinen Formel (**IV**), auch noch die Zwischenprodukte der allgemeinen Formeln (**V**) und (**VI**) im Reaktionsgemisch enthalten sein, die unter den gegebenen Reaktionsbedingungen zum Produkt der allgemeinen Formel (**IV**) weiter reagieren.

Die Verbindungen der allgemeinen Formel (**II**) und (**III**) sind kommerziell erhältlich. Die Verbindungen der allgemeinen Formel (**III**) können als *E*- oder *Z*-Isomer eingesetzt werden. Unter den Reaktionsbedingungen findet eine Isomerisierung zwischen *Z*- und *E*-Isomeren statt. Bevorzugt wird das Z-Isomer umgesetzt.

Die Verbindungen der allgemeinen Formel (**I**) weisen ein Stereozentrum auf. Folglich liegt das Produkt in Form eines Racemates vor.

### Zyklisierung:

Die Zyklisierung ist aus dem Stand der Technik bekannt und wird dort mit NaH oder Natriumpulver durchgeführt (Helv. Chim. Acta 1959, 1177; WO 2016/205633). Diese Reagenzien sind nicht für eine großtechnische Synthese geeignet, da der sichere Umgang mit ihnen im großen Maßstab schwierig ist.

Die Ausbeute des erfindungsgemäße liegt höher (> 30%) als bei der im Stand der Technik beschriebenen Reaktion mit NaH bzw. Natriumpulver (< 30%). Außerdem ist die Reaktion wegen der Alkoholat-Base auch großtechnisch einsetzbar.

Vorteilhaft für das Erzielen einer hohen Ausbeute ist das langsame Zugeben von der Alkoholat-Base, z.B. durch Zudosieren.

Außerdem kann das Gleichgewicht der Reaktion zum Produkt verschoben werden, wenn der als Lösungsmittel benutzte und zusätzlich bei der Zyklisierung entstehende Alkohol destillativ entfernt wird. Die Ausbeute steigt.

Das molare Verhältnis von **M**O-**Z** bezogen auf Verbindungen der allgemeinen (**II**) beträgt 0,8 bis 3 Äquivalente, bevorzugt 0,9 bis 1,2 Äquivalente.

Das molare Verhältnis von **Z**-OH bezogen auf Verbindungen der allgemeinen Formel (**II**) beträgt 1 bis 10 Äquivalente, bevorzugt 4 bis 7 Äquivalente.

Das molare Verhältnis von Verbindungen der allgemeinen Formel (**II**) zu Verbindungen der allgemeinen Formel (**III**) beträgt 0,8 bis 3 Äquivalente, bevorzugt 0,9 bis 1,2 Äquivalente.

Die Temperatur kann in einem breiten Bereich variiert werden und ist z.B. abhängig vom Lösungsmittel. Sie liegt für die Reaktion bevorzugt bei 15 bis 70°C, besonders bevorzugt bei 40 bis 60°C.

Die Reaktion wird gewöhnlich in einem Lösungsmittel durchgeführt, bevorzugt in Anisol, THF, Toluol, Xylol oder Me-THF. Das Lösungsmittel kann Z-OH enthalten. Bevorzugt handelt es sich um wasserfreies ("trockenes" oder absolutes) Lösungsmittel.

### Dealkoxycarbonylierung (Organic Reactions, Vol 81):

Während mit Schwefelsäure in Wasser (s. Helv. Chim. Acta 1959, 1177; WO 2016/205633) die Esterspaltung/Decarboxylierung zu 4-Oxotetrahydrofurancarbonsäure stattfindet, kann unter nicht hydrolytischen, z.B. wasserfreien Reaktionsbedingungen, der Ester in Verbindungen der allgemeinen Formel (**I**) bestehen bleiben, sodass der zusätzliche Schritt der erneuten Veresterung, der im Stand der Technik mit CH₂N₂ vorgenommen wird, entfallen kann. Dadurch entfallen auch die im Stand der Technik notwendigen zahlreichen Extraktionen der 4-Oxotetrahydrofurancarbonsäure, die schwer aus Wasser zu isolieren ist.

Die Ausbeute kann in der Folge wesentlich gesteigert werden (>95% gegenüber 75% mit Schwefelsäure im Stand der Technik). Auf toxische Reagenzien, wie z.B. Diazomethan, kann verzichtet werden.

Das Reagenz (s. Tabelle 1) wird im Überschuss eingesetzt gegebenenfalls in Kombination mit einem Lösungsmittel. Bevorzugt wird das Reagenz auch als Lösungsmittel verwendet.

Die Temperatur für die Reaktion ist abhängig vom Reagenz bzw. Lösungsmittel.

In Tabelle 1 sind beispielhaft einige dieser Reaktionsbedingungen aufgeführt, ohne sie jedoch auf diese zu beschränken.

**Tabelle 1: Dealkoxycarbonylierung**

| **Bedingungen (Reagenz = Lösungsmittel)** | **Temp/°C** | **Zeit/Stunden** |
|---|---|---|
| Essigsäure | 118 | 6 |
| Essigsäure/Trifluoressigsäure | 115-118 | 25 |
| Propionsäure | 140 | 18 |
| B(OH)₃ | 145-175 | 4 |

### Beispiele

Die vorliegende Erfindung wird anhand der nachfolgenden Beispiele näher erläutert, ohne die Erfindung dabei auf diese einzuschränken.

### Messverfahren

Die Produkte wurden mittels ¹H-NMR charakterisiert.

### Beispiel 1

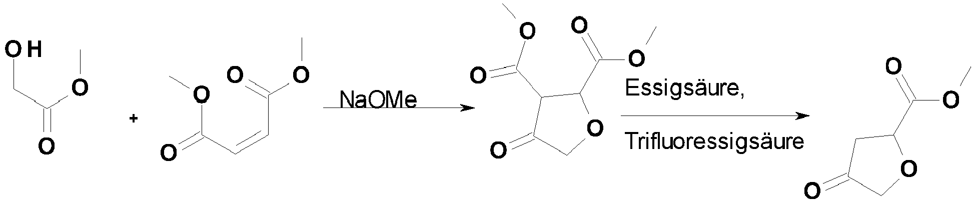

### Methyl-4-oxotetrahydrofuran-2,3-dicarboxylat

356 g Methylglycolat (3.95 mol) und 541 g Dimethylmaleat (3.75 mol) werden zusammen mit 2,1751 Anisol in einen Kessel mit Heiz/Kühlmantel (61) gegeben. Die Mischung wird auf 49°C erwärmt und dann werden 783 g 30%iges Natriummethylat (NaOMe = NaOMethyl) in Methanol über 6 Stunden hizugefügt. Während der ersten Minuten steigt die Innentemperatur auf 55°C und bleibt dann bei 50°C. Nach Zugabe von ca. 30% der Base wird das Reaktionsgemisch trübe und ein Feststoff fällt aus. Nach dem Ende der Zugabe der Base liegt eine bräunliche, noch gut rührbare Suspension vor. Das Reaktionsgemisch wird auf 20°C abgekühlt und über Nacht gerührt. Am nächsten Tag wird das Reaktionsgemisch auf 50°C (55°C Heizmantel) erwärmt und innerhalb von 6 Stunden werden 849 g Methanol destillativ entfernt, und zwar bei einem Druck von 360 mbar (am Anfang) bis 60 mbar (zum Schluss). Dann wird die Reaktionsmischung auf 20°C abgekühlt und über Nacht gerührt. Diese Reaktionsmischung wird dann zu einer Mischung aus verdünnter Schwefelsäure, hergestellt aus 2.373 1 Wasser und 225 g konzentrierter Schwefelsäure (2.17 mol) gegeben, die sich in einem Kessel mit Heiz/Kühlmantel (61) befindet, und zwar bei 10°C. Das Reaktionsgemisch wird für 2 Stunden bei 10 bis 12°C gerührt, dann wird auf 20°C erwärmt. Anschließend wird das 2-Phasen-Gemisch getrennt und die wässrige Phase wird mit Anisol extrahiert. Die vereinten organischen Phasen werden eingeengt auf 990g.

### Methyl-4-oxotetrahydrofuran-2-carboxylat

Das Öl aus dem vorhergehenden Reaktionsschritt wird in 1200 ml Essigsäure gegeben. Es werden 67.6 g Trifluoressigsäure hinzugefügt und das Reaktionsgemisch wird für 24 Stunden bei 115 bis 118°C gerührt. Dann wird die Essigsäure abdestilliert und das Produkt wird destillativ gereinigt. Das Produkt ist ein Öl (350 g, 66% Ausbeute).
NMR-Daten
¹H-NMR (600MHz, DMSO-d6): δ (ppm) = 4.97 (dd, *J* = 8.8, 5.1 Hz, 1H), 4.02 (d, *J* = 16.7 Hz, 1H), 3.99 (d, *J* = 16.7 Hz, 1H), 3.69 (s, 3H), 2.88 (dd, *J* = 18.2, 8.8 Hz, 1H), 2.60 (dd, *J* = 18.2, 5.1 Hz, 1H).

**Tabelle 2: Vergleich der Ausbeute**

| | **Reaktionsbedingungen** | **Gesamtausbeute** |
|---|---|---|
| **Beispiel Nr.1** | NaOMe/Essigsäure/Trifl uoressigsäure | **66%** |
| **Vergleichsbeispiel:** Helv. Chim. Acta 1959, 1177 | Natriumpulver, Schwefelsäure, Wasser, CH₂N₂ | **23%** |

## Patentansprüche

1. Verfahren zur Herstellung von Verbindungen der allgemeinen Formel (**I**) 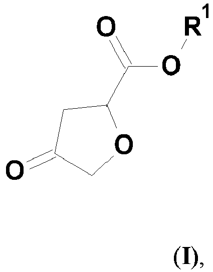 worin
R¹ (C₁-C₄)-Alkyl ist,
**dadurch gekennzeichnet, dass** Verbindungen der allgemeinen Formel (**II**) 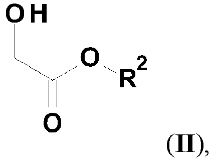 worin
R² (C₁-C₄)-Alkyl ist,
mit Verbindungen der allgemeinen Formel (**III**) 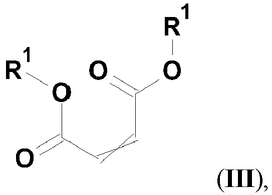 worin R¹ wie oben definiert ist,
durch Zugabe von **M**O-**Z** in **Z**-OH,
worin
M für ein Alkalimetallion steht und
Z (C₁-C₄)-Alkyl, ausgenommen *t*Butyl ist,
Zyklisierungsprodukte mit der allgemeinen Formel (**IV**) ergeben 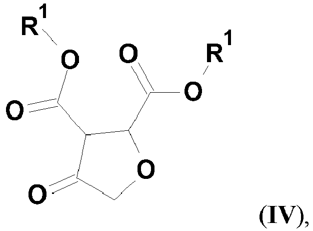 worin R¹ wie oben definiert ist,
welche unter nicht hydrolytischen Bedingungen eine Dealkoxycarbonylierung unterlaufen und zu Verbindungen der allgemeinen Formel (**I**) reagieren.

2. Verfahren nach Anspruch 1, **dadurch gekennzeichnet, dass** die Restedefinitionen für die Verbindungen der allgemeinen Formeln (**I**), (**II**), (**III**), (**IV**), **M**O-**Z** und **Z**-OH die folgenden sind:
R¹ steht für Ethyl oder Methyl,
R² steht für Ethyl oder Methyl,
M steht für Natrium oder Kalium,
Z steht für Ethyl oder Methyl.

3. Verfahren nach Anspruch 1, **dadurch gekennzeichnet, dass** die Restedefinitionen für die Verbindungen der allgemeinen Formeln (**I**), (**II**), (**III**), (**IV**), **M**O-**Z** und **Z**-OH die folgenden sind:
R¹ steht für Methyl,
R² steht für Methyl,
M steht für Natrium,
Z steht für Methyl.

4. Verfahren nach einem der Ansprüche 1 bis 3, **dadurch gekennzeichnet, dass** neben dem Zyklisierungsprodukt mit der allgemeinen Formel (**IV**) auch die Intermediate (**V**) und (**VI**) entstehen, die ebenfalls zu Verbindungen der Formel (**IV**) weiter reagieren können.

5. Verfahren nach einem der Ansprüche 1 bis 4, **dadurch gekennzeichnet, dass** der Alkohol **Z-**OH während der Zyklisierungsreaktion destillativ entfernt wird.

6. Verfahren nach einem der Ansprüche 1 bis 5, **dadurch gekennzeichnet, dass** die Zyklisierung bei 15 bis 70 °C durchgeführt wird.

7. Verfahren nach einem der Ansprüche 1 bis 5, **dadurch gekennzeichnet, dass** die Zyklisierung bei 40 bis 60 °C durchgeführt wird.

8. Verfahren nach einem der Ansprüche 1 bis 7, **dadurch gekennzeichnet, dass** das Lösungsmittel bei der Zyklisierung Anisol, THF, Toluol, Xylol oder Me-THF.

9. Verfahren nach Anspruch 8, **dadurch gekennzeichnet, dass** das Lösungsmittel auch **Z**-OH enthält, wobei **Z** wie in den Ansprüchen 1 bis 3 definiert ist.

10. Verfahren nach einem der Ansprüche 8 bis 9, **dadurch gekennzeichnet, dass** für die Zyklisierung wasserfreies Lösungsmittel verwendet wird.

11. Verfahren nach einem der Ansprüche 1 bis 10, **dadurch gekennzeichnet, dass** das Z-Isomer der Verbindung der allgemeinen Formel (**III**) eingesetzt wird.

12. Verfahren nach einem der Ansprüche 1 bis 11, **dadurch gekennzeichnet, dass M**O-**Z** in **Z-**OH zudosiert wird.

13. Verfahren nach einem der Ansprüche 1 bis 12, **dadurch gekennzeichnet, dass** NaOMethyl bei der Zyklisierung verwendet wird.

14. Verfahren nach einem der Ansprüche 1 bis 13, **dadurch gekennzeichnet, dass** das Reagenz bzw. Lösungsmittel der Dealkoxycarbonylierung Essigsäure ist oder aus einer Mischung aus Essigsäure und Trifluoressigsäure besteht.
